# EUROPEAN PATENT APPLICATION

(11) **EP 3 903 879 A1**
(43) Date of publication of application: **03.11.2021**
(21) Application number: 18945203.0
(22) Date of filing: 29.12.2018
(51) Int. Cl.: A61N 1/39

(54) **DEFIBRILLATION METHOD, AUTOMATIC EXTERNAL DEFIBRILLATOR AND COMPUTER READABLE MEDIUM**

(71) Applicant: Shenzhen Mindray Bio-Medical Electronics Co., Ltd., Shenzhen, Guangdong 518057 (CN)
(72) Inventor: WANG, Qi, Shenzhen, Guangdong 518057 (CN); CHEN, Dabing, Shenzhen, Guangdong 518057 (CN); LI, Zhiwei, Shenzhen, Guangdong 518057 (CN); LI, Liya, Shenzhen, Guangdong 518057 (CN)
(74) Representative: KIPA AB
(86) International application number: PCT/CN2018/125852
(87) International publication number: WO 2020/133525

(57) **Abstract**

A defibrillation method, an automatic external defibrillator and a computer readable medium. The defibrillation method comprises the following steps: acquiring a power-on instruction and completing initialization according to the power-on instruction (S 110); determining whether an electrode pad of an automatic external defibrillator is attached to a target subject (S 120); once the electrode pad is attached to the target subject, sensing a first ECG signal corresponding to the heart activity of the target subject (S130); analyzing the first ECG signal so as to determine whether the target subject meets an electric shock condition (S140); when the target subject meets the electric shock condition and an electric shock instruction is received, performing electrotherapy on the target subject (S 150); prior to "analyzing the first ECG signal so as to determine whether the target subject meets an electric shock condition" (S 140), the defibrillation method further comprises: starting to charge a charging circuit in the automatic external defibrillator (S10).

## Description

### TECHNICAL FIELD

The present application relates to the technical field of medical instruments, and in particular, to a defibrillation method, an automated external defibrillator, and a computer-readable medium.

### BACKGROUND

Heart diseases such as cardiac arrest are one of main causes of human death. About 85% to 90% of patients with cardiac arrest undergo ventricular fibrillation in an early stage. The main way to treat ventricular fibrillation is to use an automated external defibrillator (AED) to perform electric shock defibrillation on patients. According to research, for every minute of delay in defibrillation, a patient's survival rate is reduced by 7% to 10%. Therefore, how to perform timely defibrillation when a patient is in an initial episode is of great importance for saving the patient's life. However, a conventional automated external defibrillator takes a long time from detecting that a patient meets an electric shock defibrillation condition to performing electric shock defibrillation on the patient.

### SUMMARY

The present application provides a defibrillation method applied to an automated external defibrillator, the defibrillation method comprising:
acquiring a power-on instruction and completing initialization according to the power-on instruction;
determining whether electrode pads of the automated external defibrillator are attached to a target subject;
sensing a first ECG signal corresponding to a heart activity of the target subject when the electrode pads are attached to the target subject;
analyzing the first ECG signal to determine whether the target subject meets an electric shock condition; and
performing electrotherapy on the target subject when the target subject meets the electric shock condition and an electric shock instruction is received,
wherein the defibrillation method further comprises: before the "analyzing the first ECG signal to determine whether the target subject meets an electric shock condition": charging a charging circuit in the automated external defibrillator.

The present application further provides an automated external defibrillator, wherein the automated external defibrillator comprises:
an instruction acquisition module configured to acquire a power-on instruction and complete initialization according to the power-on instruction;
a determination module configured to determine whether electrode pads of the automated external defibrillator are attached to a target subject;
a sensing module configured to sense a first ECG signal corresponding to a heart activity of the target subject when the electrode pads are attached to the target subject,
wherein the determination module is further configured to analyze the first ECG signal to determine whether the target subject meets an electric shock condition; and
a discharging module configured to perform electrotherapy on the target subject by means of a discharging circuit of the automated external defibrillator when the target subject meets the electric shock condition and an electric shock instruction is received; and
wherein the automated external defibrillator further comprises: a control module and a charging module, wherein the control module is configured to control, before the determination module analyzes the first ECG signal to determine whether the target subject meets an electric shock condition, the charging module to charge the charging circuit in the automated external defibrillator.

The present application further provides an automated external defibrillator, the automated external defibrillator comprising electrode pads, a sensor, a processor, and a memory, wherein the sensor senses a first ECG signal corresponding to a heart activity of a target subject by means of the electrode pads when the electrode pads are attached to the target subject; and the memory stores a computer-readable program, and when the computer-readable program is read and executed by the processor, the defibrillation method is performed.

The present application further provides a computer-readable medium, wherein the computer-readable medium is used to store a computer program, and when the computer-readable program is executed, the defibrillation method is performed.

Compared with the prior art, the defibrillation method and the automated external defibrillator provided in the present application make it possible to charge the charging circuit before the first ECG signal is analyzed to determine whether the target subject meets the electric shock condition, which can store the time for charging the charging circuit after it is determined that the target subject meets the electric shock condition and an electric shock instruction is received, such that after the target subject meets the electric shock condition and the electric shock instruction is received, electrotherapy can be quickly performed on the target subject, thereby saving the time taken for the automated external defibrillator from detecting that the target subject meets the electric shock defibrillation condition to performing electric shock defibrillation on the target subject, and thus facilitating saving the life of the target subject.

### BRIEF DESCRIPTION OF THE DRAWINGS

In order to illustrate the structural features and effects of the present application more clearly, they will be described in detail below with reference to the drawings and specific embodiments. Apparently, the drawings in the following description are some of the embodiments of the present application, and those of ordinary skill in the art would also have been able to obtain other drawings according to these drawings without involving any inventive effort.
FIG. 1 is a schematic diagram of a circuit structure of an automated external defibrillator to which a defibrillation method provided according to the present application is applied.
FIG. 2 is a schematic flowchart of a defibrillation method provided according to an embodiment of the present application.
FIG. 3 is a schematic diagram of processes comprised in S120 in the defibrillation method shown in FIG. 2.
FIG. 4 is a schematic diagram of processes comprised in S130 in the defibrillation method shown in FIG. 2.
FIG. 5 is a schematic diagram of processes comprised in S150 in the defibrillation method shown in FIG. 2.
FIG. 6 is a schematic flowchart of a defibrillation method provided according to another embodiment of the present application.
FIG. 7 is a schematic structural diagram of modules in an automated external defibrillator provided according to an embodiment of the present application.
FIG. 8 is a schematic circuit diagram of a sensing module in the automated external defibrillator shown in FIG. 7.
FIG. 9 is a schematic structural diagram of modules in an automated external defibrillator provided according to another embodiment of the present application.
FIG. 10 is a schematic structural diagram of modules in an automated external defibrillator provided according to a further embodiment of the present application.
FIG. 11 is a schematic structural diagram of modules in an automated external defibrillator provided according to a further embodiment of the present application.

### DESCRIPTION OF THE PREFERRED EMBODIMENTS

The technical solutions in the embodiments of the present application will be clearly and completely described below with reference to the accompanying drawings in the embodiments of the present application. Apparently, the described embodiments are some, rather than all, of the embodiments of the present application. Based on the embodiments in the present application, all other embodiments that would be derived by those of ordinary skill in the art without involving any inventive effort shall all fall within the scope of protection of the present application.

"Embodiment" mentioned herein means that a specific feature, structure, or characteristic described with reference to the embodiment may be included in at least one embodiment of the present application. The phrase at various locations in the specification does not necessarily refer to the same embodiment, or an independent or alternative embodiment exclusive of other embodiments. Those skilled in the art understand, in explicit and implicit manners, that an embodiment described herein may be combined with another embodiment.

In order to make the technical solutions provided in the embodiments of the present application clearer, the above solutions are described in detail below with reference to the drawings.

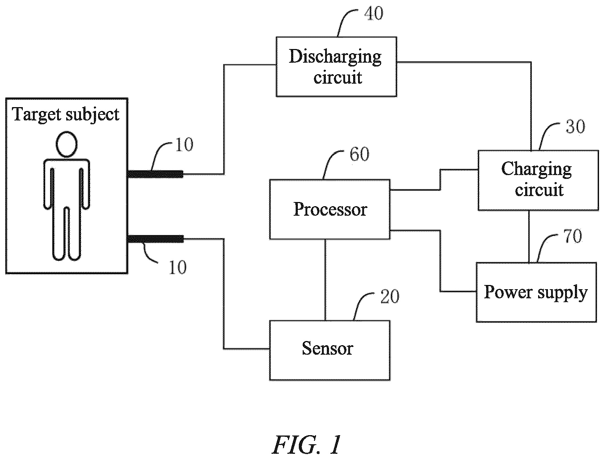

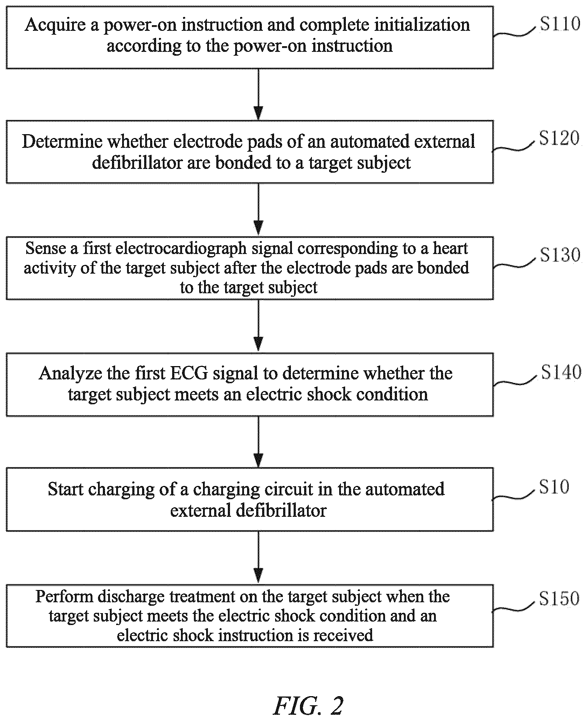

Referring to FIGS. 1 and 2 together, FIG. 1 is a schematic diagram of a circuit structure of an automated external defibrillator to which a defibrillation method provided according to the present application is applied; and FIG. 2 is a schematic flowchart of a defibrillation method provided according to an embodiment of the present application. An automated external defibrillator 1 comprises a pair of electrode pads 10, a sensor 20, a charging circuit 30, a discharging circuit 40, and a power supply 50. The functions and principles of various components in the automated external defibrillator are described in conjunction with the defibrillation method of the present application.

The defibrillation method provided in the embodiment of the present application comprises, but is not limited to, steps S110, S120, S130, S140, S150, and S10, and each step is described in detail as follows.

S110: A power-on instruction is acquired, and initialization is completed according to the power-on instruction.

S120: It is determined whether the electrode pads 10 of the automated external defibrillator 1 are attached to a target subject.

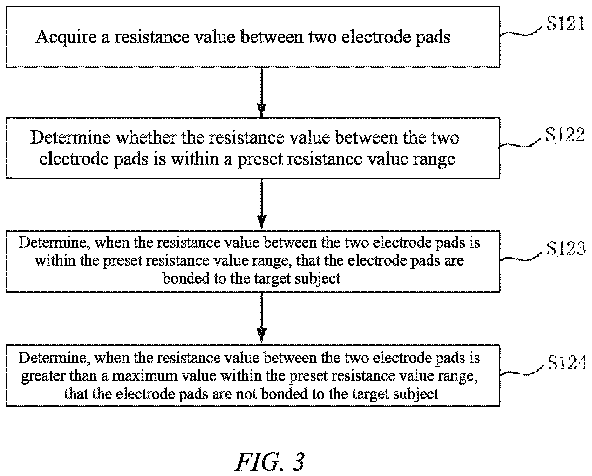

Specifically, also referring to FIG. 3, FIG. 3 is a schematic diagram of processes comprised in S120 in the defibrillation method shown in FIG. 2. S120 comprises S121, S122, S123, and S124. S121, S122, S123, and S124 are described in detail as follows.

S121: A resistance value between two electrode pads 10 is acquired.

S122: It is determined whether the resistance value between the two electrode pads 10 is within a preset resistance value range.

S123: When the resistance value between the two electrode pads 10 is within the preset resistance value range, it is determined that the electrode pads 10 are attached to the target subject.

S124: When the resistance value between the two electrode pads 10 is greater than a maximum value within the preset resistance value range, it is determined that the electrode pads 10 are not attached to the target subject.

S130: A first electrocardiograph (ECG) signal corresponding to a heart activity of the target subject is sensed after the electrode pads 10 are attached to the target subject. Specifically, when the electrode pads 10 are attached to the target subject, the sensor 20 senses the heart activity of the target subject and obtains the electrocardiograph signal corresponding to the heart activity of the target subject, so as to obtain the first ECG signal.

Generally, when the resistance value between the two electrode pads 10 is within the preset resistance value range, that is, the resistance value between the two electrode pads 10 is greater than or equal to a minimum value within the preset resistance value range or less than or equal to the minimum value within the preset resistance value range, it is determined that the electrode pads 10 are attached to the target subject and at a proper position; when the resistance value between the two electrode pads 10 is greater than a maximum value within the preset resistance value range, it is determined that the electrode pads 10 are not attached to the target subject; and when the resistance value between the two electrode pads 10 is less than the minimum value within the preset resistance value range, it is determined that the two electrode pads 10 are short-circuited or the distance between the two electrode pads 10 attached to the target subject is too close. Generally, the maximum value of the preset resistance value range is greater than an upper limit value of a human body's resistance by a preset margin. In this embodiment, the preset resistance value range is greater than 10 ohms and less than 600 ohms, that is, the maximum value of the preset resistance value range is 600 ohms, and the minimum value of the preset resistance value range is 10 ohms.

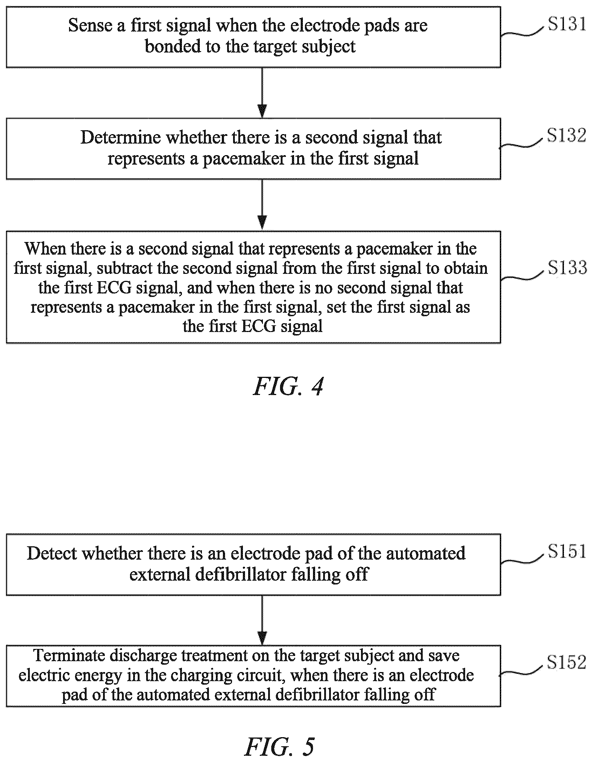

Specifically, also referring to FIG. 4, FIG. 4 is a schematic diagram of processes comprised in S130 in the defibrillation method shown in FIG. 2. S130 comprises S131, S132, and S133. S131, S132, and S133 are described in detail as follows.

S131: A first signal when the electrode pads 10 are attached to the target subject is sensed.

S132: It is determined whether there is second signal representing a pacemaker in the first signal.

S133: When there is second signal representing a pacemaker in the first signal, the second signal is subtracted from the first signal to obtain the first ECG signal, and when there is no second signal representing a pacemaker in the first signal, the first signal is set as the first ECG signal.

When the target subject wears a pacemaker, the pacemaker will interfere with the determination as to whether the target subject meets an electric shock condition. Therefore, in this embodiment, the interference of the pacemaker can be removed to avoid misjudgment.

S140: The first ECG signal is analyzed to determine whether the target subject meets an electric shock condition.

S150: Electrotherapy is performed on the target subject when the target subject meets the electric shock condition and an electric shock instruction is received.

In an embodiment, FIG. 5 is a schematic diagram of processes comprised in S150 in the defibrillation method shown in FIG. 2. Before "S150 of performing electrotherapy on the target subject", the defibrillation method further comprises S151 and S153, and S151 and S153 are described in detail as follows.

S151: It is detected whether there is an electrode pad 10 of the automated external defibrillator 1 falling off from the target subject.

S153: Electrotherapy on the target subject is terminated and electric energy in the charging circuit 30 is stored, when there is an electrode pad 10 of the automated external defibrillator 1 falling off from the target subject.

In this embodiment, when there is an electrode pad 10 of the automated external defibrillator 1 falling off from the target subject, electrotherapy on the target subject is terminated to prevent a defibrillation signal released by the discharging circuit 40 of the automated external defibrillator 1 by means of the electrode pad 10 from being delivered to a rescuer who is rescuing the target subject, so as to prevent defibrillation energy from causing harm to the rescuer. Further, saving the electric energy in the charging circuit 30 can reduce the energy consumption of the power supply 50 for charging the charging circuit 30.

Before "S 140 of analyzing the first ECG signal to determine whether the target subject meets an electric shock condition", the defibrillation method further comprises S10 of starting charging of the charging circuit 30 in the automated external defibrillator 1. For the convenience of illustration, in FIG. 1, S10 is marked before S150 and after S140. It can be understood that S10 only needs to be before S150, and is not limited to being before S150 and after S140.

The defibrillation method further comprises: prohibiting the electrotherapy to the target subject and saving the power in the charging circuit 30, when the target subject meets the electric shock condition but no electric shock instruction is received within a preset time period.

In this embodiment, when no electric shock instruction is received within the preset time period, prohibiting the electrotherapy to the target subject and saving the power in the charging circuit 30 can reduce the energy consumption of the power supply 50 for charging the charging circuit 30.

Further, after "S10 of starting charging of a charging circuit 30 in the automated external defibrillator 1", the defibrillation method further comprises: charging the charging circuit 30 in the automated external defibrillator 1 to a first preset amount of power, wherein the first preset amount of power is greater than or equal to an amount of power required to perform electrotherapy on the target subject.

The defibrillation method in the present application is applied to the automated external defibrillator 1, and the automated external defibrillator 1 comprises electrode pads 10 and a sensor 20. Generally, there are two electrode pads 10. When the automated external defibrillator 1 is used, the two electrode pads 10 are attached to the target subject. For example, the electrode pads 10 are attached to, but not limited to, the chest of the target subject. When the electrode pads 10 are attached to the target subject, the sensor 20 can sense an ECG signal of the heart activity of the target subject by means of the electrode pads 10, so as to obtain a first ECG signal. In this embodiment, the target subject may be an adult or a child. When the first ECG signal is obtained, the first ECG signal is analyzed to determine whether the target subject meets an electric shock condition. For example, when it is determined according to the first ECG signal that a heart rhythm of the target subject is characterized by at least one of ventricular fibrillation, ventricular tachycardia, and ventricular flutter, it may be determined that the target subject meets the electric shock condition. When it is determined according to the first ECG signal that the heart rhythm of the target subject is characterized by any one of bradycardia, electromechanical dissociation, accelerated idioventricular rhythm, and normal heart rhythm, it may be determined that the target subject does not meet the electric shock condition.

When the electrode pads 10 are attached to the target subject, and when the target subject meets the electric shock condition and an electric shock instruction is received, electrotherapy is performed on the target subject. In an embodiment, when the target subject meets the electric shock condition, an electric shock instruction is automatically triggered. In another embodiment, the automated external defibrillator 1 comprises a discharge button, and when the discharge button is pressed, an electric shock instruction is triggered. Specifically, when the target subject meets the electric shock condition, an alarm unit of the automated external defibrillator 1 sends out prompt information, which is used to give a prompt that the target subject is allowed to be subjected to an electric shock, and an operator can press the discharge button according to the prompt information, so as to trigger the electric shock instruction.

Referring to FIG. 1, the automated external defibrillator 1 further comprises a charging circuit 30 and a discharging circuit 40. The charging circuit 30 is configured to receive and store electric energy, and when the target subject meets the electric shock condition, the energy stored in the charging circuit 30 is loaded onto the electrode pad 10 by means of the discharging circuit 40 and transferred to the target subject. In an embodiment, the charging circuit 30 and the discharging circuit 40 may also be integrated into a charging and discharging module. The charging and discharging module can not only receive a charging signal and store energy, but also can release the stored energy. In an embodiment, the automated external defibrillator 1 comprises a power supply 50, and the power supply 50 is configured to provide electric energy to the charging circuit 30. The power supply 50 may be a disposable battery or a rechargeable battery.

After an electric shock instruction is triggered, the discharging circuit 40 in the automated external defibrillator 1 delivers a defibrillation signal to the target subject by means of the electrode pads 10, and the defibrillation signal is used to facilitate the restoring of the heart rhythm of the target subject to normal. Generally, the defibrillation signal is a high-voltage pulse signal.

When the automated external defibrillator 1 performs electrotherapy on the target subject, a certain amount of power is required, and when the charging circuit 30 is charged until an amount of power required to perform electrotherapy on the target subject is met, a certain charging time is required. Then, the defibrillation method in the embodiment of the present application makes it possible to charge the charging circuit 30 of the automated external defibrillator 1 before "S140 of analyzing the first ECG signal to determine whether the target subject meets an electric shock condition", which can store the time for charging the charging circuit 30 after it is determined that the target subject meets the electric shock condition and an electric shock instruction is received, such that after the target subject meets the electric shock condition and the electric shock instruction is received, electrotherapy can be quickly performed on the target subject, thereby saving the time taken for the automated external defibrillator 1 from detecting that the target subject meets the electric defibrillation condition to performing electric shock defibrillation on the target subject, and thus facilitating saving the life of the target subject.

Further, before "analyzing the first ECG signal to determine whether the target subject meets an electric shock condition", the defibrillation method of the present application comprises charging the charging circuit 30 in the automated external defibrillator 1 to a first preset amount of power, wherein the first preset amount of power is greater than or equal to an amount of power required to perform electrotherapy on the target subject. Therefore, when the target subject meets the electric shock condition and the electric shock instruction is received, there is a sufficient amount of power to treat the target subject, which further shortens the time taken for the defibrillator from power-on to the electrotherapy to the target subject.

Still further, when the first preset amount of power is greater than the amount of power required to perform electrotherapy on the target subject, after "S150 of performing electrotherapy on the target subject when the target subject meets the electric shock condition and an electric shock instruction is received", the defibrillation method further comprises S160.

S160: The remaining power in the charging circuit 30 is stored.

Further, after S160, the defibrillation method further comprises S170.

S170: The charging circuit 30 is charged on the basis of saving the remaining power in the charging circuit 30. Preferably, in S170, the charging circuit 30 is charged to a second preset amount of power on the basis of saving the remaining power in the charging circuit 30. The second preset amount of power is greater than or equal to the amount of power required to perform electrotherapy on the target subject.

In this embodiment, when the first preset amount of power is greater than the amount of power required to perform electrotherapy on the target subject, the remaining power in the charging circuit 30 is stored after the electrotherapy is performed on the target subject, and the charging circuit 30 is charged on the basis of the remaining power, such that the time for charging the charging circuit 30 to the second preset amount of power can be shortened, and the energy consumption of the power supply 50 for charging the charging circuit 30 can be reduced.

Further, after S170, the defibrillation method further comprises S180, S182, and S 184. S180, S182, and S 184 are described in detail as follows.

S180: A second ECG signal corresponding to the heart activity of the target subject is sensed.

S182: The second ECG signal is analyzed to determine whether the target subject meets the electric shock condition.

S184: Electrotherapy is performed on the target subject again, when the target subject meets the electric shock condition.

After electrotherapy is performed on the target subject in S150, the second ECG signal corresponding to the heart activity of the target subject continues to be sensed, and when the target subject meets the electric shock condition, electrotherapy is performed on the target subject again.

Further, in other embodiments, while the second ECG signal corresponding to the heart activity of the target subject is sensed, the charging circuit 30 is charged on the basis of saving the remaining power in the charging circuit 30. That is, after S160, the defibrillation method comprises: S172, S174, and S176.

S172: While the second ECG signal corresponding to the heart activity of the target subject is sensed, the charging circuit 30 is charged on the basis of saving the remaining power in the charging circuit 30.

S174: The second ECG signal is analyzed to determine whether the target subject meets the electric shock condition.

S176: When the target subject meets the electric shock condition, and when the amount of power in the charging circuit 30 is greater than or equal to an amount of power required to perform electrotherapy on the target subject, electrotherapy is performed on the target subject again.

According to the defibrillation method in this embodiment, while the second ECG signal corresponding to the heart activity of the target subject is sensed, the charging circuit 30 starts to be charged on the basis of saving the remaining power in the charging circuit 30, thereby shortening the time required to perform electrotherapy on the target subject again.

Further, the defibrillation method further comprises: S152: It is prohibited to discharge charge to the target subject and the power in the charging circuit 30 is stored, when the target subject meets the electric shock condition but no electric shock instruction is received within a preset time period.

In this embodiment, when the target subject meets the electric shock condition but no electric shock instruction is received within a preset time period, no charge is discharged to the target subject and the power in the charging circuit 30 is stored, that is, the charging circuit 30 does not release energy, such that the energy consumption of the power supply 50 for charging the charging circuit 30 can be reduced. The preset time period is counted from the time when it is determined that the target subject meets the electric shock condition. The preset time period may be, but is not limited to, within 30 seconds from the time when it is determined that the target subject meets the electric shock condition.

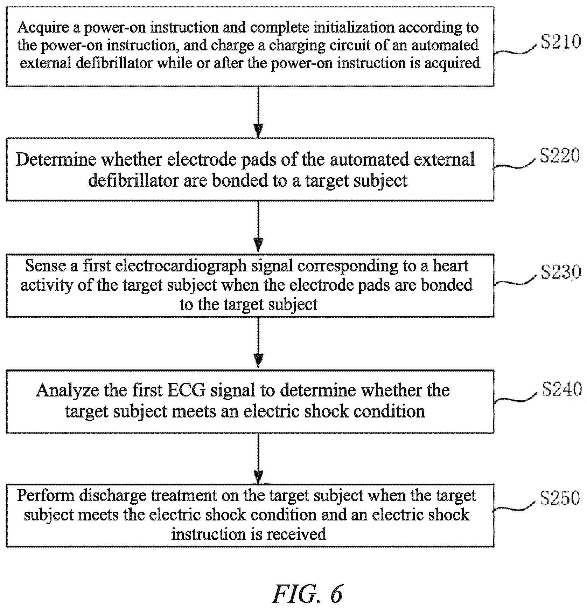

Referring to FIG. 6, FIG. 6 is a schematic flowchart of a defibrillation method provided according to another embodiment of the present application. The defibrillation method comprises, but is not limited to, S210, S220, S230, S240, and S250, and the steps are described in detail as follows. The defibrillation method provided in this embodiment is basically the same as the defibrillation method provided in the above embodiments, except that in this embodiment, the charging circuit 30 of the automated external defibrillator 1 is charged while or after the power-on instruction is acquired. For the convenience of illustration, the defibrillation method provided in this embodiment is described as follows. Although the defibrillation method is described as comprising S210, S220, S230, S240, and S250 as an example in this embodiment and related drawings, it can be understood that for the same steps in this embodiment as those in the above embodiments, reference may be made to the above description, which will not be described in detail herein.

S210: A power-on instruction is acquired and initialization is completed according to the power-on instruction, and the charging circuit 30 of the automated external defibrillator 1 is charged while or after the power-on instruction is acquired.

S220: It is determined whether the electrode pads 10 of the automated external defibrillator 1 are attached to a target subject.

S230: A first electrocardiograph (ECG) signal corresponding to a heart activity of the target subject is sensed when the electrode pads 10 are attached to the target subject.

S240: The first ECG signal is analyzed to determine whether the target subject meets an electric shock condition.

S250: Electrotherapy is performed on the target subject when the target subject meets the electric shock condition and an electric shock instruction is received.

In an embodiment of the present application, charging the charging circuit 30 of the automated external defibrillator 1 while acquiring the power-on instruction can further store the time taken for the automated external defibrillator 1 from detecting that the target subject meets an electric shock defibrillation condition to performing electric shock defibrillation on the target subject, thereby facilitating saving the life of the target subject.

Specifically, the time required from acquiring the power-on instruction to analyzing the first ECG signal and then to determining that the target subject meets the electric shock condition is a first time, and the time required from charging the charging circuit 30 of the automated external defibrillator 1 from zero electric energy until the amount of power required to perform electrotherapy on the target subject is met is a second time. The time taken from acquiring the power-on instruction to determining that the target subject meets the electric shock condition and then to charging the charging circuit 30 until the amount of power required to perform electrotherapy on the target subject is met is a third time, wherein the third time is equal to the first time plus the second time. Generally, the second time is less than the first time. For example, the second time is 8 seconds, the first time is 10 seconds, and the third time is 18 seconds. Charging the charging circuit 30 of the automated external defibrillator 1 while acquiring the power-on instruction in this embodiment can store time and thus facilitate saving the life of the target subject, compared with determining that the target subject meets the electric shock condition and then charging the charging circuit 30.

Still further, in an embodiment, while or after the power-on instruction is acquired, the charging circuit 30 of the automated external defibrillator 1 is charged and the charging thereof is stopped when it is determined that the target subject meets the electric shock condition, that is, the charging circuit 30 is charged in the first time; because the first time is greater than the second time, for the same charging voltage, charging the charging circuit 30 of the automated external defibrillator 1 while acquiring the power-on instruction can make it possible for the charging circuit 30 to store more power, and there will be some remaining power even after electrotherapy is performed on the target subject; and when the charging circuit 30 continues to be charged on the basis of the remaining power, such that the electric energy required to perform electrotherapy on the target subject is met again, the charging time can be stored.

Still further, in an embodiment, a charging voltage for charging the charging circuit 30 of the automated external defibrillator 1 in the first time is less than a voltage for charging the charging circuit 30 of the automated external defibrillator 1 in the second time, such that the electric energy charged to the charging circuit 30 of the automated external defibrillator 1 in the first time is exactly equal to the electric energy required to perform electrotherapy on the target subject. In this embodiment, the requirement for levels of the power supply 50 and components in the charging circuit 30 and the discharging circuit 40 in the automated external defibrillator 1 can be reduced, which facilitates improving the reliability of the automated external defibrillator 1. For example, when the power supply 50 is a 12 V disposable battery, for a disposable battery with a voltage of 12 V, when the charging time is the second time (8 seconds), the battery is required to have a load capacity of 3.25 A; and when the charging time is the first time (10 seconds), the battery is required to have a load capacity of 2.6 A. Therefore, in this embodiment, the requirements for the power supply 50 and the components in the charging circuit 30 and the discharging circuit 40 of the automated external defibrillator 1 are lower. In the case where the power supply 50 is at the same level as the components in the charging circuit 30 and the discharging circuit 40 in the automated external defibrillator 1, the stability and reliability of the automated external defibrillator 1 to which the defibrillation method is applied can be improved in this embodiment.

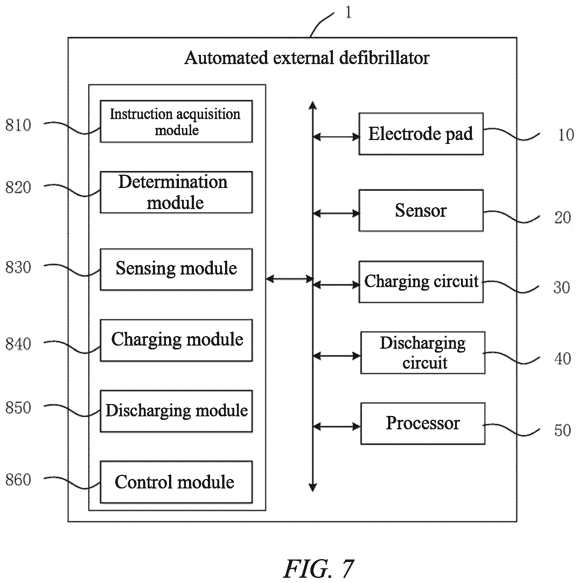

The present application further provides an automated external defibrillator 1. The automated external defibrillator 1 of the present application is described below in conjunction with the defibrillation method described above. Also referring to FIG. 7, FIG. 7 is a schematic structural diagram of modules in an automated external defibrillator provided according to an embodiment of the present application. The automated external defibrillator 1 comprises an instruction acquisition module 810, a determination module 820, a sensing module 830, a charging module 840, a discharging module 850, and a control module 860. The instruction acquisition module 810, the determination module 820, the sensing module 830, the charging module 840, the discharging module 850, and the control module 860 may be solidified in the automated external defibrillator 1 in the form of a software program or firmware, or may be stored in a memory 70 of the automated external defibrillator 1, and a processor 60 of the automated external defibrillator 1 controls the execution of individual functional modules. The detailed working principles of individual modules are described as follows.

The instruction acquisition module 810 is configured to acquire a power-on instruction and complete initialization according to the power-on instruction.

The determination module 820 is configured to determine whether electrode pads 10 of the automated external defibrillator 1 are attached to a target subject.

The sensing module 830 is configured to sense a first ECG signal corresponding to a heart activity of the target subject after the electrode pads 10 are attached to the target subject.

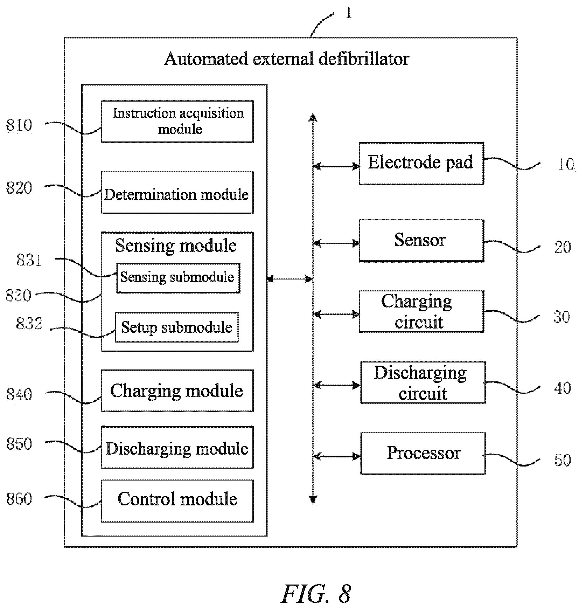

Specifically, referring to FIG. 8, FIG. 8 is a schematic circuit diagram of a sensing module in the automated external defibrillator shown in FIG. 7. The sensing module 830 further comprises a sensing submodule 831 and a setup submodule 831. The sensing submodule 831 is configured to sense a first signal when the electrode pads 10 are attached to the target subject. The determination module 820 is configured to determine whether there is second signal representing a pacemaker exists in the first signal. The setup submodule 831 is configured to subtract, when there is second signal representing a pacemaker exists in the first signal, the second signal from the first signal to obtain the first ECG signal, and set, when there is no second signal representing a pacemaker in the first signal, the first signal as the first ECG signal.

The determination module 820 is further configured to analyze the first ECG signal to determine whether the target subject meets an electric shock condition.

The discharging module 850 is configured to perform electrotherapy on the target subject by means of a discharging circuit 40 of the automated external defibrillator 1 when the target subject meets the electric shock condition and an electric shock instruction is received.

The control module 860 is configured to control, before the determination module 820 analyzes the first ECG signal to determine whether the target subject meets the electric shock condition, the charging module 840 to start charging of the charging circuit 30 in the automated external defibrillator 1.

Further, the control module 860 controls, before the determination module 820 analyzes the first ECG signal to determine whether the target subject meets the electric shock condition, the charging module 840 to start charging of the charging circuit 30 in the automated external defibrillator 1 to a first preset amount of power, wherein the first preset amount of power is greater than or equal to an amount of power required to perform electrotherapy on the target subject.

Further, the control module 860 controls the charging module 840 to start charging of the charging circuit 30 of the automated external defibrillator 1 when or after the instruction acquisition module 810 acquires the power-on instruction.

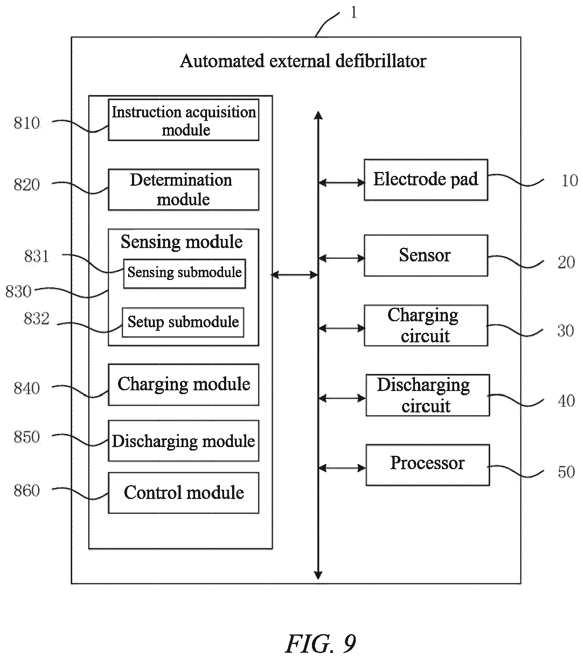

Still further, referring to FIG. 9, FIG. 9 is a schematic structural diagram of modules in an automated external defibrillator provided according to another embodiment of the present application. The structure of the automated external defibrillator 1 provided in this embodiment is basically the same as that of the automated external defibrillator 1 provided in FIG. 8 and related descriptions thereof, except that in this embodiment, the discharging module 850 stores the remaining power in the charging circuit 30 after performing electrotherapy on the target subject.

Still further, after the discharging module 850 performs electrotherapy on the target subject, the charging module 840 further charges the charging circuit 30 on the basis of saving the remaining power in the charging circuit 30. Optionally, after the discharging module 850 performs electrotherapy on the target subject, the charging module 840 charges the charging circuit 30 to a second preset amount of power on the basis of saving the remaining power in the charging circuit 30.

Still further, after the discharging module performs electrotherapy on the target subject by means of the discharging module 850 of the automated external defibrillator 1, the sensing module 830 further senses a second ECG signal corresponding to the heart activity of the target subject; the determination module 820 further analyzes the second ECG signal to determine whether the target subject meets an electric shock condition; and the discharging module 850 further performs electrotherapy on the target subject again, when the target subject meets the electric shock condition.

Further, the discharging module 850 is prohibited to discharge charge to the target subject and stores the power in the charging circuit 30, when the target subject meets the electric shock condition but no electric shock instruction is received within a preset time period.

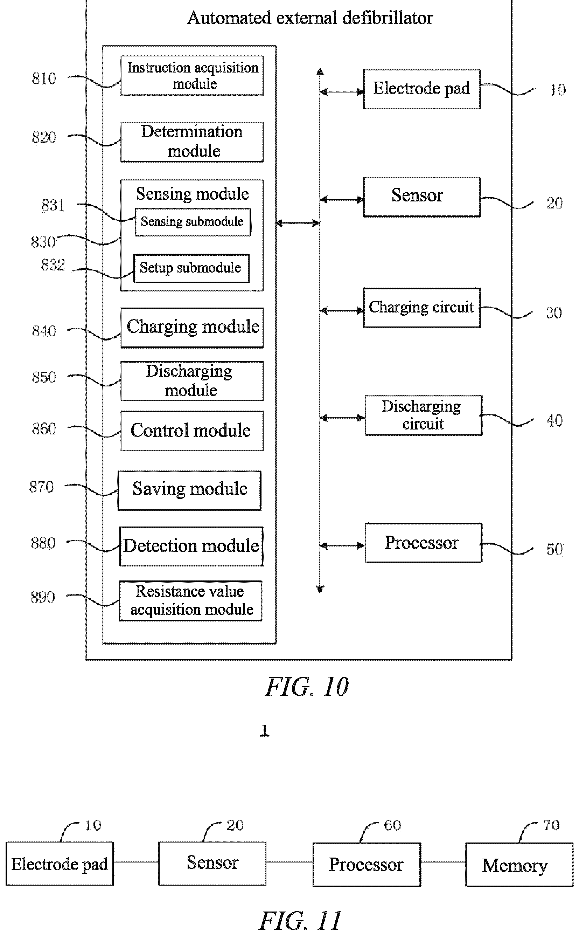

Further, referring to FIG. 10, FIG. 10 is a schematic structural diagram of modules in an automated external defibrillator provided according to a further embodiment of the present application. The automated external defibrillator 1 further comprises: a detection module 880. The detection module 880 is configured to detect whether there is an electrode pad 10 of the automated external defibrillator 1 falling off from the target subject before electrotherapy is performed on the target subject. The discharging module 850 terminates the electrotherapy to the target subject and stores the energy in the charging circuit 30, when there is an electrode pad 10 of the automated external defibrillator 1 falling off from the target subject.

Further, the automated external defibrillator 1 further comprises: a resistance value acquisition module 890 configured to acquire a resistance value between two electrode pads 10. The determination module 820 further determines whether the resistance between the two electrode pads 10 is within a preset resistance value range. When the resistance value between the two electrode pads 10 is within the preset resistance value range, it is determined that the electrode pads 10 are attached to the target subject.

It can be understood that, for further functions of individual modules in the automated external defibrillator 1 described in the above embodiment, reference may be made to the above description of specific steps in the defibrillation method, which will not be described in detail herein. For example, the instruction acquisition module 810 is configured to acquire a power-on instruction and complete initialization according to the power-on instruction. Therefore, for the instruction acquisition module 810, reference may be made to the related description of the steps of acquiring a power-on instruction and completing initialization according to the power-on instruction when the defibrillation method is described above.

The present application further provides an automated external defibrillator 1. Referring to FIG. 11, FIG. 11 is a schematic structural diagram of modules in an automated external defibrillator provided according to a further embodiment of the present application. The automated external defibrillator 1 comprises electrode pads 10, a sensor 20, a processor 60, and a memory 70, wherein the sensor 20 senses a first ECG signal corresponding to a heart activity of a target subject by means of the electrode pads 10 when the electrode pads 10 are attached to the target subject; and the memory 70 stores a computer-readable program, and when the computer-readable program is read and executed by the processor 60, a defibrillation method of any one of the above embodiments is performed.

In addition, the present application further provides a computer-readable medium, wherein the computer-readable medium is used to store a computer program, and when the computer-readable program is executed, a defibrillation method of any one of the above embodiments is performed.

The embodiments of the present application have been described above in detail, specific examples are used herein to explain the principles and implementations of the present application, and the description of the above embodiments is only intended to help understand the method of the present application and a core idea thereof. In addition, for those skilled in the art, changes can be made to the specific implementations and the range of application thereof based on the idea of the present application. In summary, the content of the specification should not be construed as limiting the present application.

## Claims

1. A defibrillation method applied to an automated external defibrillator, comprising:
acquiring a power-on instruction and completing initialization according to the power-on instruction;
determining whether electrode pads of the automated external defibrillator are attached to a target subject;
sensing a first ECG signal corresponding to a heart activity of the target subject after the electrode pads are attached to the target subject;
analyzing the first ECG signal to determine whether the target subject meets an electric shock condition; and
performing electrotherapy on the target subject when the target subject meets the electric shock condition and an electric shock instruction is received,
**characterized in that** before the "analyzing the first ECG signal to determine whether the target subject meets an electric shock condition", the defibrillation method further comprises: starting charging of a charging circuit in the automated external defibrillator.

2. The defibrillation method of claim 1, **characterized in that** after the "starting charging of a charging circuit in the automated external defibrillator", the defibrillation method further comprises: charging the charging circuit in the automated external defibrillator to a first preset amount of power, wherein the first preset amount of power is greater than or equal to an amount of power required to perform electrotherapy on the target subject.

3. The defibrillation method of claim 1, **characterized in that** the charging of the charging circuit of the automated external defibrillator is started while or after the power-on instruction is acquired.

4. The defibrillation method of claim 2, **characterized in that** the first preset amount of power is greater than the amount of power required to perform electrotherapy on the target subject, and
after the "performing electrotherapy on the target subject when the target subject meets the electric shock condition and an electric shock instruction is received", the defibrillation method further comprises:
saving the remaining power in the charging circuit.

5. The defibrillation method of claim 4, **characterized in that** after the "performing electrotherapy on the target subject", the defibrillation method further comprises:
charging the charging circuit on the basis of the remaining power stored in the charging circuit.

6. The defibrillation method of claim 5, **characterized in that** after the "charging the charging circuit on the basis of the remaining power stored in the charging circuit", the defibrillation method further comprises:
sensing a second ECG signal corresponding to the heart activity of the target subject;
analyzing the second ECG signal to determine whether the target subject meets the electric shock condition; and
performing electrotherapy on the target subject again, when the target subject meets the electric shock condition.

7. The defibrillation method of claim 1, **characterized in that** the defibrillation method further comprises:
prohibiting the electrotherapy to the target subject and saving the power in the charging circuit, when the target subject meets the electric shock condition but no electric shock instruction is received within a preset time period.

8. The defibrillation method of claim 1, **characterized in that** before the "performing electrotherapy on the target subject", the defibrillation method further comprises:
detecting whether there is at least one of the electrode pads falling off from the target subject; and
terminating the electrotherapy to the target subject and saving the energy in the charging circuit, when there is at least one of the electrode pads falling off from the target subject.

9. The defibrillation method of claim 1, **characterized in that** the "determining whether electrode pads of the automated external defibrillator are attached to a target subject" comprises:
acquiring a resistance value between two electrode pads;
determining whether the resistance value between the two electrode pads is within a preset resistance value range; and
determining, when the resistance value between the two electrode pads is within the preset resistance value range, that the electrode pads are attached to the target subject.

10. The defibrillation method of claim 1, **characterized in that** the "sensing a first ECG signal corresponding to a heart activity of the target subject" comprises:
sensing a first signal when the electrode pads are attached to the target subject;
determining whether there is second signal representing a pacemaker exists in the first signal; and
subtracting, when second signal representing a pacemaker exists in the first signal, the second signal from the first signal to obtain the first ECG signal, and setting, when the second signal representing a pacemaker is not in the first signal, the first signal as the first ECG signal.

11. An automated external defibrillator comprising:
an instruction acquisition module configured to acquire a power-on instruction and complete initialization according to the power-on instruction;
a determination module configured to determine whether electrode pads of the automated external defibrillator are attached to a target subject;
a sensing module configured to sense a first ECG signal corresponding to a heart activity of the target subject after the electrode pads are attached to the target subject;
the determination module is further configured to analyze the first ECG signal to determine whether the target subject meets an electric shock condition; and
a discharging module configured to perform an electrotherapy on the target subject by a discharging circuit of the automated external defibrillator when the target subject meets the electric shock condition and an electric shock instruction is received; and
**characterized in that** the automated external defibrillator further comprises: a control module and a charging module, the control module is configured to control the charging module to start charging of a charging circuit in the automated external defibrillator before the determination module analyzes the first ECG signal to determine whether the target subject meets the electric shock condition.

12. The automated external defibrillator of claim 11, **characterized in that** the control module is configured to control the charging module to charge the charging circuit in the automated external defibrillator to a first preset amount of power, the first preset amount of power is greater than or equal to an amount of power required to perform the electrotherapy on the target subject.

13. The automated external defibrillator of claim 11, **characterized in that** the control module is configured to control the charging module to start charging of the charging circuit of the automated external defibrillator when or after the instruction acquisition module acquires the power-on instruction.

14. The automated external defibrillator of claim 11, **characterized in that** the discharging module is configured to control store the remaining power in the charging circuit after performing the electrotherapy on the target subject.

15. The automated external defibrillator of claim 14, **characterized in that** after the discharging module performs the electrotherapy on the target subject, the charging module is further configured to charges the charging circuit on the basis of the remaining power stored in the charging circuit.

16. The automated external defibrillator of claim 15, **characterized in that** after the discharging module performs the electrotherapy on the target subject by the discharging circuit of the automated external defibrillator,
the sensing module is further configured to sense a second ECG signal corresponding to a heart activity of the target subject;
the determination module is further configured to analyze the second ECG signal to determine whether the target subject meets the electric shock condition; and
the discharging module is further configured to perform an electrotherapy on the target subject again, when the target subject meets the electric shock condition.

17. The automated external defibrillator of claim 11, **characterized in that** the discharging module is prohibited to perform the electrotherapy on the target subject and the power is stored in the charging circuit, when the target subject meets the electric shock condition but no electric shock instruction is received within a preset time period.

18. The automated external defibrillator of claim 11, **characterized in that** the automated external defibrillator further comprises:
a detection module configured to detect whether at least one of the electrode pads detaches from the target subject before the electrotherapy is performed on the target subject; and
wherein the discharging module is configured to terminate the electrotherapy to the target subject and stores the energy in the charging circuit, when at least one of the electrode pads detaches from the target subject.

19. The automated external defibrillator of claim 11, **characterized in that** the automated external defibrillator further comprises:
a resistance value acquisition module configured to acquire a resistance value between two of the electrode pads; and
the determination module is further configured to:
determine whether the resistance between the two electrode pads is within a preset resistance value range; and
determine that the two electrode pads are attached to the target subject, when the resistance value between the two electrode pads is within the preset resistance value range.

20. The automated external defibrillator of claim 11, **characterized in that** the sensing module comprises: a sensing submodule and a setup submodule,
the sensing submodule is configured to sense a first signal when the electrode pads are attached to the target subject;
the determination module is further configured to determine whether second signal representing a pacemaker exists in the first signal; and
the setup submodule is configured to subtract the second signal from the first signal to obtain the first ECG signal, when the second signal representing a pacemaker exists in the first signal, and,
set the first signal as the first ECG signal, when the second signal representing a pacemaker is not in the first signal.

21. An automated external defibrillator, **characterized in that** the automated external defibrillator comprises electrode pads, a sensor, a processor, and a memory, wherein the sensor senses a first ECG signal corresponding to a heart activity of a target subject by means of the electrode pads when the electrode pads are attached to the target subject; and the memory stores a computer-readable program, and when the computer-readable program is read and executed by the processor, a defibrillation method of any one of claims 1 to 10 is performed.

22. A computer-readable medium, **characterized in that** the computer-readable medium is used to store a computer program, and when the computer-readable program is executed, a defibrillation method of any one of claims 1 to 10 is performed.
